# EUROPÄISCHE PATENTSCHRIFT

(11) **EP 1 438 940 B1**
(45) Veröffentlichungstag und Bekanntmachung des Hinweises auf die Patenterteilung: **06.02.2008**
(21) Anmeldenummer: 03027977.2
(22) Anmeldetag: 05.12.2003
(51) Int. Cl.: A61F 7/02

(54) **Kühl- und Wärmegerät**
Cooling and heating device
Appareil réfrigérant et réchauffant

(30) Priorität: 15.01.2003 DE 20300554 U
(43) Veröffentlichungstag der Anmeldung: 21.07.2004
(73) Patentinhaber: Kall Schanktechnik GmbH, 35708 Haiger-Weidelbach (DE)
(72) Erfinder: Leufgen, Martin, 53881 Euskirchen (DE)
(74) Vertreter: KEIL & SCHAAFHAUSEN Patentanwälte

(56) Entgegenhaltungen:
- EP-A- 0 144 877
- WO-A-01/54635
- DE-A- 3 925 461
- US-A- 4 338 944
- US-A- 4 844 072
- US-A- 5 411 541
- US-A- 5 948 012
- US-A1- 2002 138 033

## Beschreibung

Die vorliegende Erfindung betrifft eine Kühl- und Wärmegerät mit einem Wärmetauscherelement, durch welches über Fluidleitungen und eine Pumpe ein in einem Tank gespeichertes Medium zirkulierbar ist, wobei dem Tank eine Wärmequelle und -senke zum Temperieren des in dem Tank gespeicherten Mediums zugeordnet ist.

Es ist bekannt, bei Knochenbrüchen, Verstauchungen, Prellungen, Schwellungen oder anderen Verletzungen oder Erkrankungen diese mit Eisbeuteln oder anderen Kühlelementen zu behandeln. Dies ist mit hohem Arbeitsaufwand verbunden, da Eisbeutel oder derartige Kühlelemente zum schnellen Einsatz in einem Kühlfach temperiert vorgehalten werden müssen. Da die Kühlwirkung derartiger Kühlelemente nicht lange anhält, müssen diese in kurzen Zeitabständen gegen andere temperierte Kühlelemente ausgetauscht werden. Zudem besteht bei der Verwendung von Eisbeuteln oder Kühlelementen der Nachteil, dass die Temperierung eines Körperteils eines Patienten nur mit der besonders niedrigen Temperatur des Eisbeutels möglich ist. Dies ist für einige Anwendungsfälle ungünstig.

Es wird daher in der US 5,895,418 ein mobiles Kühl- und Wärmegerät der eingangs genannten Art vorgeschlagen. Das Wärmetauscherelement ist dabei als ein Kissen ausgebildet, das auf ein zu behandelndes Körperteil eines Patienten aufgelegt werden kann. Dieses Kissen wird je nach Betriebsmodus von einem erwärmten oder gekühlten Medium durchströmt. Eine Veränderung der Temperatur des Mediums während des Betriebes ist bei diesem Gerät nicht möglich.

In der DE 296 09 945 U1 wird eine Vorrichtung zur Kältetherapie beschrieben, welche einen Wasserbehälter mit einem zugeordnetem Kältesatz und eine Wasserpumpe zur Förderung des gekühlten Wassers durch eine Kühlkompresse aufweist. An der Vorrichtung ist ein Bedienfeld angeordnet, an welchem die Durchflussmenge, die Kühltemperatur und die Behandlungsdauer eingestellt und überwacht werden kann.

Die DE 195 35 640 A1 schlägt eine Vorrichtung zur Erzeugung rascher Temperaturwechsel für die Schmerzforschung vor, die einen an ein Körperteil anlegbaren Kontaktkopf umfasst, durch den Fluide unterschiedlicher Temperatur geleitet werden können.

Weiter offenbaren auch die DE 39 25 461 A1 sowie die DE 296 23 224 U1 Einrichtungen zum Temperieren von bspw. oberflächennahen Bereichen von Organismen.

Einige Anwendungsfälle machen es erforderlich, dass zusätzlich zu einer Temperierung von Körperteilen eines Patienten auch bspw. eine Druckbeaufschlagung der Körperteile erfolgt. Dies ist jedoch mit keinem der aus den oben genannten Druckschriften bekannten Gerät möglich. In der DE 82 16 834 U1 wird eine Einrichtung zum Überwärmen bzw. zur Kältebehandlung von Körperteilen beschrieben, bei welcher temperierte Luft mittels eines Kompressors durch einen den Körperteil überdeckenden Überzug geleitet wird. Hierdurch wölbt sich der Überzug auf, ohne dass jedoch eine nennenswerte Druckbeaufschlagung des von dem Überzug umschlossenen Körperteils erfolgt.

Aus der US 5,411,541 sowie der US 4,338,944 sind Kühl- und Wärmegeräte bekannt, die zusätzlich zu einer Temperierung eines Körperteils eines Patienten auch eine Druckbeaufschlagung ermöglichen. Hierzu weist eine von einem temperierten Medium durchströmte Manschette oder dergleichen auch eine beispielsweise mit Druckluft beaufschlagbare Kammer auf. Diese bekannten Geräte ermöglichen jedoch nur sehr eingeschränkt eine individuell auf einen Patienten abgestimmte Behandlung durch Druck- bzw. Temperaturbeaufschlagung.

Aufgabe der vorliegenden Erfindung ist es daher ein Kühl- und Wärmegerät der eingangs genannten Art bereitzustellen, bei welchem in Abhängigkeit der zu behandelnden Verletzung oder dgl. eine individuelle Behandlung mit einer bei Bedarf veränderbaren, vorgegebenen Temperatur- und/oder Druckbeaufschlagung automatisiert erfolgen kann.

Diese Aufgabe wird erfindungsgemäß im Wesentlichen durch ein Gerät mit den Merkmalen des Anspruchs 1 gelöst. Der Erfindung liegt dabei der Gedanke zugrunde, dass für verschiedene Therapiefälle, d. h. für unterschiedliche Verletzungen und/oder für unterschiedliche zu behandelnde Körperteile eines Patienten, verschiedene Behandlungstemperaturen erforderlich sind. Diese können mit dem Kühl- und Wärmegerät dadurch erzeugt werden, dass die Durchflussmenge des Mediums durch das Wärmetauscherelement, welches mit dem zu behandelnden Körperteil eines Patienten in Kontakt gebracht wird, oder die Temperierung des Mediums selbst variiert wird. Dies erfolgt über die Steuerungseinrichtung die zur Regelung der Durchflussmenge und/oder zur Regelung der Temperatur des Mediums mit der Pumpe und/oder der Wärmequelle und -senke verbunden ist. Falls erforderlich, kann mit dem erfindungsgemäßen Kühl- und Wärmegerät gleichzeitig eine Massage oder dgl. durch eine Druckbeaufschlagung erfolgen. Dies kann entweder durch eine Komprimierung des zu behandelnden Körperteils mit konstantem Druck oder durch einen sich verändernden, bspw. pulsierenden Druck in dem Wärmetauscherelement erreicht werden. Die Therapiemöglichkeiten mit diesem Kühl- und Wärmegerät werden dadurch erhöht und optimiert.

Weiter ist es vorgesehen, dass die Steuerungseinrichtung derart ausgebildet ist, dass die Temperatur des Mediums nach einem voreinstellbaren Temperaturverlauf variiert werden kann. So ist es bspw. möglich, eine zu behandelnde Körperstelle eines Patienten zunächst durch Beaufschlagung des Wärmetauscherelementes mit Medium mit einer Temperatur von bspw. 15°C für einige Minuten nur leicht abzukühlen, dann durch Absenkung der Temperatur des Mediums auf z. B. 6°C kurzzeitig ein Kältereiz zu erzeugen und die zu behandelnde Körperstelle dann wieder zu erwärmen. Die Möglichkeit der frei wählbaren Temperatursequenzen ermöglicht dabei einen neuen therapeutischen Ansatz, der bisher insbesondere mit derart einfachem Aufwand nicht zu erreichen war.

Zur Vermeidung von Verletzungen hat es sich als vorteilhaft erwiesen, dass der Steuerungseinrichtung eine Überwachungseinrichtung zugeordnet wird, so dass die Steuerungseinrichtung zumindest den Betrieb der Pumpe bei Erreichen einer voreinstellbaren Minimal- oder Maximaltemperatur des Mediums und/oder bei Überschreiten einer voreinstellbaren Zeitspanne einer Kälte- oder Wärmebehandlung abschaltet. Diese Kontrolle durch die Überwachungseinrichtung ist vorzugsweise unabhängig von der Regelung des Temperaturverlaufs durch die Steuerungseinrichtung. Bei dem erfindungsgemäßen Kühl- und Wärmegerät lassen sich auf diese Weise übermäßig lange Kälte- bzw. Wärmephasen verhindern, so dass das Gerät eigenständig die zulässigen Minimal- oder Maximaltemperaturen überwacht und bei Auftritten einer Fehlfunktion durch einen Bedienungsfehler oder einen Gerätefehler das Gerät selbsttätig abschaltet.

Erfindungsgemäß ist als Wärmequelle und -senke ein thermoelektrisches Peltier-Element vorgesehen. Dieses erzeugt bei Anschluss an eine Gleichspannung auf einer Seite Kälte und auf der anderen Seite Wärme, um so das Medium in dem Tank zu temperieren. Über einen der Wärmequelle und -senke zugeordneten Lüfter, der bspw. ebenfalls über die Steuerungseinrichtung regelbar ist, kann die erzeugte Wärme- an die Umgebung abgeführt werden. Die Peltier-Elemente sind dabei bspw. mit Hilfe von Spannern an dem Tank befestigt, so dass die Temperierung des Tanks im Kontaktverfahren erfolgt. Zum Einfüllen des Mediums kann der Tank von außen zugänglich sein.

Die eingesetzte Pumpe ist vorzugsweise eine selbstansaugende Umwälzpumpe, durch die kontinuierlich temperiertes Medium aus dem Tank dem Wärmetauscherelement zugeleitet werden kann. Dabei kann auch die Pumpe im Betrieb in dem Wärmetauscherelement einen Druck erzeugen, so dass ein bspw. von dem Wärmetauscherelement umschlossener Körperteil eines Patienten während der Behandlung komprimiert wird. Diese Komprimierung des zu behandelnden Gewebes hat zusätzlich zu der Temperierung einen positiven Effekt, der die Behandlung zusätzlich unterstützt.

Um das Kühl- und Wärmegerät leicht transportabel mobil einsetzten zu können, sind die Pumpe, der Tank und die Wärmequelle und -senke nach einer bevorzugten Ausführungsform der Erfindung in einem gemeinsamen Gehäuse aufgenommen. Das Gehäuse kann dabei bspw. aus Edelstahl oder aus Kunststoff bestehen.

In Weiterbildung dieses Gedankens ist es vorgesehen, in oder an dem Gehäuse eine insbesondere wiederaufladbare Stromquelle anzubringen. Das Kühl- und Wärmegerät ist auf diese Weise unabhängig von einer externen Energieversorgung und eignet sich bspw. für den mobilen oder stationären Einsatz in einem Krankenwagen, zuhause oder in einer Arztpraxis.

Alternativ oder zusätzlich hierzu kann in oder an dem Gehäuse ein Netzgerät vorgesehen sein, welches insbesondere zum Betrieb mit verschiedenen Spannungen und Gleich- und/oder Wechselstrom ausgerüstet ist. Durch den Einsatz dieses speziellen Netzgerätes lässt sich das erfindungsgemäße Kühl- und Wärmegerät bspw. sowohl an die Energieversorgung in einem Kraftfahrzeug als auch an das Hausstromnetz anschließen.

Die Fluidleitungen sind vorzugsweise auf ihren dem Wärmetauscherelement zugewandten Seite und/oder tankseitig mit Schnellspannkupplungen versehen. Auf diese Weise lassen sich die für den Behandlungszweck optimalen Wärmetauscherelemente schnell gegeneinander austauschen und über die Fluidleitungen mit dem Tank verbinden.

Um eine Fehlfunktion des erfindungsgemäßen Kühl- und Wärmegerätes zu verhindern, ist es erforderlich, dass eine ausreichende Menge an Medium in dem Tank vorgehalten wird. Zur Kontrolle des Niveaus des Mediums kann hierzu der Tank mit einem Füllstandsröhrchen verbunden sein, über das das Niveau des Mediums in dem Tank abgelesen werden kann. Alternativ oder zusätzlich hierzu kann in oder an dem Tank eine Anzeigeeinheit vorgesehen sein, die bspw. über LEDs die Anzeige des Niveaus des Mediums in dem Tank ermöglicht. Diese Anzeige des Niveaus des Mediums in dem Tank kann dabei entweder durch die Anzahl der aufleuchtenden LEDs und/oder durch deren Farbe erfolgen.

Der Energieverbrauch des erfindungsgemäßen Kühl- und Wärmegerätes kann dadurch minimiert werden, dass der Tank und/oder die Fluidleitungen zumindest teilweise mit einer Wärmeisolierung versehen sind.

Um die Bedienung des Kühl- und Wärmegerätes zu erleichtern, kann der Steuerungseinrichtung eine Tastatur zur Eingabe von Betriebsparametern zugeordnet sein. Durch diese Tastatur können verschiedene Temperaturverläufe oder feste Behandlungstemperaturen sowie die Behandlungszeit einprogrammiert werden. Nach einer weiteren Ausführungsform der Erfindung können diese Betriebsparameter über eine der Steuerungseinrichtung zugeordnete insbesondere alphanumerische Anzeigeeinrichtung angezeigt werden.

Es hat sich als besonders vorteilhaft erwiesen, wenn das Wärmetauscherelement bspw. als eine Manschette ausgebildet ist, die eine insbesondere an Körperteile eines Patienten anpassbare Form hat. Selbstverständlich kann das erfindungsgemäße Kühl- und Wärmegerät auch mit Anschlüssen für mehrere Manschetten versehen sein, so dass mehrere Körperteile eines Patienten gleichzeitig behandelt werden können. Vorzugsweise sind derartige Manschetten lamellenartig mit flexiblen Leitungen aufgebaut.

Wenn das Wärmetauscherelement bzw. die Manschette eine erste Kammer, welche über die Fluidleitungen mit der Pumpe verbunden ist, und eine weitere Kammer aufweist, welche mit der weiteren Pumpeinrichtung verbunden ist, kann eine Druckbeaufschlagung einer zu behandelnden Körperstelle unabhängig von der Temperierung der Körperstelle erfolgen. Die beiden Kammern der Manschette oder dgl. liegen vorzugsweise derart übereinander, dass die dem zu behandelnden Körperteil zugewandte Kammer von dem Fluid zur Temperierung durchströmt wird, während die außenliegende Kammer der Druckbeaufschlagung dient.

Es hat sich als besonders günstig herausgestellt, dass der durch die Pumpe und/oder durch die weitere Pumpeneinrichtung in dem Wärmetauscherelement erzeugte Druck während des Betriebs definiert veränderbar ist. So kann eine pulsierende, stufenweise veränderliche oder ggf. auch konstante Komprimierung erzielt werden. Hierbei ist die Förderleistung der Pumpeneinrichtung vorzugsweise unabhängig von der Förderleistung der Pumpe regelbar.

Die Pumpeneinrichtung ist mit der Steuerungseinrichtung verbunden, so dass eine Programmierung des Druckverlaufes über den Behandlungszeitraum möglich ist.

Die Behandlung durch Druckbeaufschlagung kann vorzugsweise bei einem Druck von bis zu 200 mbar erfolgen, der durch die Pumpeneinrichtung in dem Wärmetauscherelement erzeugt wird. In Abhängigkeit des Therapiefalles ist es jedoch auch möglich, einen höheren Druck in der Kammer der Manschette oder dgl. aufzubauen.

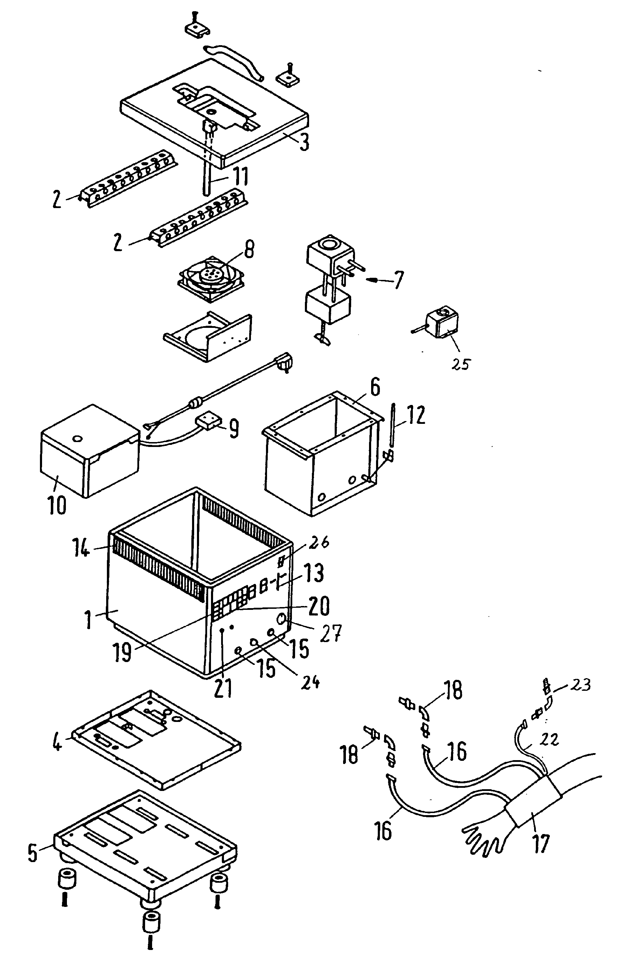

Im Folgenden wird nun die Erfindung anhand eines Ausführungsbeispiels und unter Bezugnahme auf die beigefügte Zeichnung näher erläutert.

Die einzige Figur zeigt ein erfindungsgemäßes Kühl- und Wärmegerät in Explosionsansicht.

Das in der Figur dargestellte Kühl- und Wärmegerät weist ein Gehäuse 1 aus Kunststoff oder Edelstahl auf, an welchem mittels Befestigungswinkeln 2 ein Deckel 3 sowie ein Bodenblech 4 und ein Boden 5 angebracht werden. In dem Gehäuse 1 sind ein Tank 6, eine Pumpe 7, ein Lüfter 8, ein Peltier-Element 9 und eine Elektronikbox 10 aufgenommen. Das Peltier-Element 9 wird dabei bspw. über nicht dargestellte Spanner an dem isolierten Tank 6 zur Temperierung eines in dem Tank aufgenommenen Mediums befestigt. Ein Füllanschluss 11 in dem Deckel 3 mündet zum Einfüllen des Mediums in den Tank 6. Weiter ist an dem Tank 6 ein Füllstandsröhrchen 12 vorgesehen, über welches das Niveau des Mediums in dem Tank 6 angezeigt werden kann. Hierzu ist in dem Gehäuse 1 eine Füllstandsanzeige 13 vorgesehen.

Der Lüfter 8 ist im zusammengebauten Zustand des Kühl- und Wärmegerätes in der Nähe des Peltier-Elements 9 positioniert, so dass die von dem Peltier-Element 9 erzeugte Wärme oder Kälte durch den Lüfter 8 an die Umgebung abgegeben werden kann. Hierzu sind in dem Gehäuse 1 Lüftungsschlitze 14 vorgesehen.

Weiter sind an dem Gehäuse 1 Anschlüsse 15 für Fluidleitungen 16 angeordnet, welche wiederum mit einer Manschette 17 als Wärmetauscherelement verbunden sind. Die Manschette 17 umgreift dabei ein zu behandelndes Körperteil eines Patienten, wie bspw. den in der Figur schematisch angeordneten Unterarm. Alternativ kann die Manschette 17 auch mittels Gurten oder dgl. an einem Körperteil befestigt werden oder als Kissen ausgebildet sein, welches auf ein Körperteil aufgelegt wird.

Die Fluidleitungen 16 sind zur Verbindung mit den Anschlüssen 15 tankseitig mit Stellspannkupplungen 18 versehen. Die Anschlüsse 15 des Gehäuses 1 stehen wiederum mit dem Tank 6 bzw. mit der Pumpe 7 in Verbindung, über welche Medium aus dem Tank 6 durch die Leitungen 16 in die Manschette 17 und wieder zurück in den Tank gepumpt wird.

Auf der Außenseite des Gehäuses 1 ist weiter eine Tastatur 19 und eine Anzeigeeinrichtung 20 angeordnet, die der Elektronikbox 10 zugeordnet sind. Die Elektronikbox 10 umfasst dabei eine Steuerungseinrichtung, über welche der Betrieb der Pumpe 7 sowie des Peltier-Elements 9 in Abhängigkeit der über die Tastatur 19 eingegebenen Betriebsparameter geregelt wird. Hierzu ist dem Tank 6 ein mit der Elektronikbox 10 verbundener Temperaturfühler zugeordnet. Gleichzeitig weist die Elektronikbox 10 eine Überwachungseinrichtung auf, die über die Steuerungseinrichtung den Betrieb der Pumpe 7 und/oder des Peltier-Elements 9 bei Erreichen eines voreinstellbaren Minimal- oder Maximalwerts der Temperatur des Mediums oder bei Überschreiten eines voreinstellbaren Zeitintervalls stoppt. Weiter kann in der Elektronikbox 10 auch ein Netzteil aufgenommen sein, von dem das Kühl- und Wärmegerät mit Energie versorgt wird. Vorzugsweise ist dieses Netzteil zum Betrieb mit Gleich- und Wechselstrom bei einer Spannung zwischen 12 V und 250 V geeignet.

An der Vorderseite des Gehäuses 1 sind darüber hinaus mehre Leuchtdioden (LED) 21 angeordnet, die neben dem Füllstandsröhrchen 12 ebenfalls zur Anzeige des Niveaus des Mediums in dem Tank 6 dienen.

Im Betrieb des Kühl- und Wärmegeräts wird das Medium in dem Tank 6 zunächst über das Peltier-Element 9 gekühlt oder erwärmt, wobei anfallende Abwärme durch den Lüfter 8 and die Umgebung abgegeben werden kann. Das Medium wird dann über die Pumpe 7 durch eine als Vorlaufleitung dienende Fluidleitung 16 in die Manschette 17 gepumpt, um ein zu behandelndes Körperteil eines Patienten zu temperieren. Durch den Druck des Mediums in der Manschette wird zusätzlich die zu behandelnde Körperstelle komprimiert. Über die zweite Fluidleitung 16, die als Rücklaufleitung dient, fließt das Medium in den Tank 6 zurück, in welchem es wieder temperiert wird.

Mittels der Steuerungseinrichtung lassen sich dabei verschiedene Temperaturverläufe einstellen, so dass die Temperatur des Mediums in verschiedenen Zeitintervallen unterschiedlich hoch ist. Der zur Behandlung eines Patienten eingesetzte Temperaturregelbereich liegt dabei üblicherweise zwischen 0 und 50°C.

Die Überwachungseinrichtung verhindert dabei, dass eine unzulässig hohe oder niedrige Temperatur des Mediums auftritt oder die zulässige Behandlungshöchstdauer überschritten wird. Auf diese Weise können Verletzungen des Patienten bspw. bei Fehlfunktionen des Gerätes oder Bedienfehlern vermieden werden.

Zusätzlich zu der beschriebenen Temperaturbehandlung eines Körperteils ist mit dem Kühl- und Wärmegerät auch eine Druckbeaufschlagung des Körperteils möglich, um eine massageartige Therapie durchzuführen. Hierzu ist an der Manschette 17 eine weitere Leitung 22 vorgesehen, welche über eine Schnellspannkupplung 23 mit einem entsprechenden Anschluss 24 des Gehäuses 1 verbindbar ist. Der Anschluss 24 wird von einem Luftkompressor 25 im Inneren des Gehäuses 1 gespeist, welcher über einen Schalter 26 und/oder eine Druckregulierung 27 regelbar ist. Erfindungsgemäß ist der Luftkompressor 25 auch mit der Elektronikbox 10 verbunden, um den Luftkompressor 25 durch eine entsprechende Programmierung ggf. mit einem sich über die Betriebszeit verändernden Druck zu betreiben.

Die Manschette 17 kann aus zwei nicht dargestellten Kammern aufgebaut sein, von denen die innere, an dem zu behandelnden Körperteil anliegende Kammer von dem Heiz- oder Kühlmedium durchströmt wird und mit den Fluidleitungen 16 verbunden ist. Eine zweite Kammer, welche die erste Kammer bspw. bereichsweise umgreifen kann, ist mit der Leitung 22 zur Zufuhr von Druckluft verbunden. Bei einer Druckbeaufschlagung dieser zweiten Kammer wird das zu behandelnde Körperteil in Abhängigkeit der gewählten Kompressorleistung komprimiert. Die zweite Kammer kann mit einem ggf. direkt an der Manschette 17 vorgesehenen Ablassventil verbunden sein, welches bspw. ebenfalls über die Elektronikbox 10 ansteuerbar ist.

Das Kühl- und Wärmegerät kann dabei sowohl stationär als auch mobil verwendet werden. Abweichend zu der in der Figur dargestellten Ausführungsform mit einer Kühlmanschette können bei entsprechender Auslegung des Peltier-Elements und des Tanks auch mehrere Manschetten an ein Gerät angeschlossen werden.

### Bezugszeichenliste:

- 1: Gehäuse
- 2: Befestigungswinkel
- 3: Deckel
- 4: Bodenblech
- 5: Boden
- 6: Tank
- 7: Pumpe
- 8: Lüfter
- 9: Peltier-Element (Wärmequelle und -senke)
- 10: Elektronikbox
- 11: Füllanschluss
- 12: Füllstandsröhrchen
- 13: Füllstandsanzeige
- 14: Lüftungsschlitz
- 15: Anschluss
- 16: Fluidleitung
- 17: Manschette (Wärmetauscherelement)
- 18: Schnellspannkupplung
- 19: Tastatur
- 20: Anzeigeeinrichtung
- 21: LED
- 22: Leitung
- 23: Schnellspannkupplung
- 24: Anschluss
- 25: Luftkompressor (Pumpeneinrichtung
- 26: Schalter
- 27: Druckregulierung

## Patentansprüche

1. Kühl- und Wärmegerät, insbesondere zur Temperierung zumindest von Körperteilen eines Patienten, mit einem Wärmetauscherelement (17), durch welches über Fluidleitungen (16) und eine Pumpe (7) ein in einem Tank (6) gespeichertes Medium zirkulierbar ist, wobei dem Tank (6) eine ein thermoelektrisches Peltier-Element (9) umfassende Wärmequelle und -senke (9) zum Temperieren des in dem Tank (6) gespeicherten Mediums zugeordnet ist, wobei die Pumpe (7) und/oder die Wärmequelle und -senke (9) mit einer Steuerungseinrichtung (10) zur Regelung der Durchflussmenge des Mediums durch das Wärmetauscherelement (17) und/oder zur Regelung der Temperatur des Mediums derart verbunden sind, dass zumindest die Temperatur des Mediums während des Betriebs definiert veränderbar ist, und wobei eine weitere Pumpeneinrichtung (25) im Betrieb in dem Wärmetauscherelement (17) einen Druck erzeugt, durch den ein von dem Wärmetauscherelement (17) umschlossener Gegenstand, insbesondere ein Körperteil eines Patienten, komprimiert wird, **dadurch gekennzeichnet, dass** die Steuerungseinrichtung (10) derart ausgebildet ist, dass die Temperatur des Mediums nach einem voreinstellbaren Temperaturverlauf veränderbar ist, und dass der Steuerungseinrichtung (10) eine Überwachungseinrichtung derart zugeordnet ist, dass die Steuerungseinrichtung (10) zumindest den Betrieb der Pumpe (7) in Abhängigkeit einer voreinstellbaren Minimal- oder Maximaltemperatur des Mediums und/oder in Abhängigkeit eines voreinstellbaren Zeitintervalls stoppt, und dass die weitere Pumpeneinrichtung (25) mit der Steuerungseinrichtung (10) verbunden ist, wobei der durch die Pumpe (7) und/oder durch die weitere Pumpeneinrichtung (25) in dem Wärmetauscherelement (17) erzeugte Druck während des Betriebs definiert zur Durchführung einer massageartigen Therapie veränderbar ist.

2. Kühl- und Wärmegerät nach Anspruch 1, **dadurch gekennzeichnet, dass** die Pumpe (7) eine selbstansaugende Umwälzpumpe ist.

3. Kühl- und Wärmegerät nach einem der vorhergehenden Ansprüche, **dadurch gekennzeichnet, dass** die Pumpe (7), der Tank (6) und die Wärmequelle und -senke (9) in einem gemeinsamen Gehäuse (1) aufgenommen sind.

4. Kühl- und Wärmegerät nach Anspruch 3, **dadurch gekennzeichnet, dass** in oder an dem Gehäuse (1) eine insbesondere wiederaufladbare Stromquelle vorgesehen ist.

5. Kühl- und Wärmegerät Anspruch 3, **dadurch gekennzeichnet, dass** in oder an dem Gehäuse (1) ein Netzgerät vorgesehen ist, welches insbesondere zum Betrieb mit verschiedenen Spannungen und Gleich- und/oder Wechselstrom ausgerüstet ist.

6. Kühl- und Wärmegerät nach einem der vorhergehenden Ansprüche, **dadurch gekennzeichnet, dass** das Wärmetauscherelement (17) über Schnellspannkupplungen (18) mit den Fluidleitungen (16) verbunden ist.

7. Kühl- und Wärmegerät nach einem der vorhergehenden Ansprüche, **dadurch gekennzeichnet, dass** die Fluidleitungen (16) tankseitig mit Schnellspannkupplungen (18) versehen sind.

8. Kühl- und Wärmegerät nach einem der vorhergehenden Ansprüche, **dadurch gekennzeichnet, dass** der Tank (6) mit einem Füllstandsröhrchen (12) zur Anzeige des Niveaus des Mediums in dem Tank (6) verbunden ist.

9. Kühl- und Wärmegerät nach einem der vorhergehenden Ansprüche, **dadurch gekennzeichnet, dass** in oder an dem Tank (6) eine Anzeigeeinheit, die bspw. LEDs (21) aufweist, zur Anzeige des Niveaus des Mediums in dem Tank (6) vorgesehen ist.

10. Kühl- und Wärmegerät nach einem der vorhergehenden Ansprüche, **dadurch gekennzeichnet, dass** der Tank (6) und/oder die Fluidleitungen (16) zumindest teilweise mit einer Wärmeisolierung versehen ist.

11. Kühl- und Wärmegerät nach einem der vorhergehenden Ansprüche, **dadurch gekennzeichnet, dass** der Steuerungseinrichtung (10) eine Tastatur (19) zur Eingabe von Betriebsparametern zugeordnet ist.

12. Kühl- und Wärmegerät nach einem der vorhergehenden Ansprüche, **dadurch gekennzeichnet, dass** der Steuerungseinrichtung (10) eine insbesondere alphanumerische Anzeigeeinrichtung (20) zur Anzeige von Betriebsparametern zugeordnet ist.

13. Kühl- und Wärmegerät nach einem der vorhergehenden Ansprüche, **dadurch gekennzeichnet, dass** das Wärmetauscherelement bspw. als eine Manschette (17) ausgebildet ist, die eine insbesondere an Körperteile eines Patienten anpassbare Form hat.

14. Kühl- und Wärmegerät nach einem der vorhergehenden Ansprüche, **dadurch gekennzeichnet, dass** das Wärmetauscherelement (17) eine erste Kammer, welche über die Fluidleitungen (16) mit der Pumpe (7) verbunden ist, und einer weitere Kammer aufweist, welche über eine Leitung (22) mit der weiteren Pumpeinrichtung (25) verbunden ist.

15. Kühl- und Wärmegerät nach einem der vorhergehenden Ansprüche, **dadurch gekennzeichnet, dass** die Förderleistung der Pumpeneinrichtung (25) unabhängig von der Förderleistung der Pumpe (7) regelbar ist.

16. Kühl- und Wärmegerät nach einem der vorhergehenden Ansprüche, **dadurch gekennzeichnet, dass** die Pumpeneinrichtung (25) in dem Wärmetauscherelement (17) einen Druck von bis zu 200 mbar erzeugt.

17. Kühl- und Wärmegerät nach einem der vorhergehenden Ansprüche, **dadurch gekennzeichnet, dass** der Wärmequelle und -senke (9) wenigstens ein insbesondere über die Steuerungseinrichtung (10) regelbarer Lüfter (8) zugeordnet ist.

## Claims

1. A cooling and heating apparatus, in particular for regulating the temperature at least of body parts of a patient, comprising a heat exchanger element (17), through which a medium stored in a tank (6) can be circulated via fluid lines (16) and a pump (7), wherein a heat source and heat sink (9) comprising a thermoelectric Peltier element (9) is associated with the tank (6) for regulating the temperature of the medium stored in said tank (6), and the pump (7) and/or heat source and heat sink (9) are connected to control means (10), for regulating the rate of flow of the medium through the heat exchanger element (17) and/or for regulating the temperature of the medium, in such a way that at least the temperature of the medium can be specifically adjusted during operation, and wherein in operation an additional pump device (25) generates a pressure in the heat exchanger element (17) by means of which an object enclosed by the heat exchanger element (17), in particular a body part of a patient, is compressed, **characterized in that** the control means (10) is designed in such a way that the temperature of the medium can be adjusted according to a preset temperature behaviour, and **in that** monitoring means are associated with the control means (10) in such a way that the control means (10) at least stops operation of the pump (7) as a function of a preset minimum or maximum temperature of the medium and/or as a function of a preset time interval, and **in that** the additional pump device (25) is connected to the control means (10), the pressure generated by the pump (7) and/or by the additional pump device (25) in the heat exchanger element (17) being specifically adjustable during operation for the carrying out of a massage-type therapy.

2. A cooling and heating apparatus according to Claim 1, **characterized in that** the pump (7) is a self-priming circulation pump.

3. A cooling and heating apparatus according to one of the preceding claims, **characterized in that** the pump (7), the tank (6) and the heat source and heat sink (9) are accommodated in a shared housing (1).

4. A cooling and heating apparatus according to Claim 3, **characterized in that** a specifically rechargeable power source is provided in or on the housing (1).

5. A cooling and heating apparatus according to Claim 3, **characterized in that** provided on the housing (1) is a power pack, which is specially equipped for operation at different voltages and for operation with direct current and/or alternating current.

6. A cooling and heating apparatus according to one of the preceding claims, **characterized in that** the heat exchanger element (17) is connected to the fluid lines (16) via quick-action couplings (18).

7. A cooling and heating apparatus according to one of the preceding claims, **characterized in that** the fluid lines (16) are provided at the tank end with quick-action couplings (18).

8. A cooling and heating apparatus according to one of the preceding claims, **characterized in that** the tank (6) is connected to a filling-level tube (12) for indicating the level of the medium in the tank (6).

9. A cooling and heating apparatus according to one of the preceding claims, **characterized in that** an indicator unit, involving LEDs (21) for example, is provided in or on the tank (6) for indicating the level of the medium in the tank (6).

10. A cooling and heating apparatus according to one of the preceding claims, **characterized in that** the tank (6) and/or fluid lines (16) are provided at least partly with heat insulation.

11. A cooling and heating apparatus according to one of the preceding claims, **characterized in that** a keyboard (19) for inputting operating parameters is associated with the control means (10).

12. A cooling and heating apparatus according to one of the preceding claims, **characterized in that** a specifically alphanumeric indicating device (20) for indicating operating parameters is associated with the control means (10).

13. A cooling and heating apparatus according to one of the preceding claims, **characterized in that** the heat exchanger element takes the form of a sleeve (17) for example, which has a shape which can be specifically adapted to body parts of a patient.

14. A cooling and heating apparatus according to one of the preceding claims, **characterized in that** the heat exchanger element (17) has a first chamber connected to the pump (7) via fluid lines (16), and another chamber connected to the additional pump device (25) via a line (22).

15. A cooling and heating apparatus according to one of the preceding claims, **characterized in that** the flow rate of the pump device (25) can be regulated independently of the flow rate of the pump (7).

16. A cooling and heating apparatus according to one of the preceding claims, **characterized in that** the pump device (25) generates a pressure of up to 200 mbar in the heat exchanger element (17).

17. A cooling and heating apparatus according to one of the preceding claims, **characterized in that** at least one ventilating fan (8), which can be regulated in particular via the control means (10), is associated with the heat source and heat sink (9).

## Revendications

1. Appareil réfrigérant et chauffant, en particulier permettant de tempérer au moins des parties corporelles d'un patient, comportant un élément échangeur de chaleur (17) au travers duquel un milieu emmagasiné dans un réservoir (6) peut être amené à circuler en passant par des conduites de fluide (16) et une pompe (7), une source et un dissipateur de chaleur (9) comportant un élément Peltier thermoélectrique (9) étant associés de telle sorte au réservoir (6) pour tempérer le milieu emmagasiné dans le réservoir (6), la pompe (7) et/ou la source et le dissipateur de chaleur (9) étant reliés à un dispositif de commande (10) pour réguler le débit de passage du milieu à travers l'élément échangeur de chaleur (17) et/ou pour réguler la température du milieu, qu'au moins la température du milieu peut être modifiée de manière définie durant le fonctionnement, et un autre dispositif de pompe (25) générant une pression dans l'élément échangeur de chaleur (17) durant le fonctionnement, pression grâce à laquelle un objet enfermé par l'élément échangeur de chaleur (17), à savoir en particulier une partie corporelle d'un patient, est comprimé, **caractérisé en ce que** le dispositif de commande (10) est configuré de telle sorte que la température du milieu peut être modifiée après une évolution de température pouvant être préréglée, et **en ce qu'**un dispositif de surveillance est associé au dispositif de commande (10) de telle sorte que le dispositif de commande (10) arrête au moins le fonctionnement de la pompe (7) en fonction d'une température minimale ou maximale du milieu pouvant être préréglée et/ou en fonction d'un intervalle temporel pouvant être préréglé, et **en ce que** l'autre dispositif de pompe (25) est relié au dispositif de commande (10), la pression générée par la pompe (7) et/ou par l'autre dispositif de pompe (25) dans l'élément échangeur de chaleur (17) pouvant être modifiée de manière définie pendant le fonctionnement afin de réaliser une thérapie de type massage.

2. Appareil réfrigérant et chauffant selon la revendication 1, **caractérisé en ce que** la pompe (7) est une pompe de recirculation à amorçage automatique.

3. Appareil réfrigérant et chauffant selon l'une des revendications précédentes, **caractérisé en ce que** la pompe (7), le réservoir (6) et la source et le dissipateur de chaleur (9) sont logés dans un boîtier (1) commun.

4. Appareil réfrigérant et chauffant selon la revendication 3, **caractérisé en ce qu'**une source de courant, notamment rechargeable, est prévue dans ou sur le boîtier (1).

5. Appareil réfrigérant et chauffant selon la revendication 3, **caractérisé en ce qu'**il est prévu, dans ou sur le boîtier (1), un bloc d'alimentation qui est conçu pour permettre un fonctionnement avec différentes tensions et avec du courant continu et/ou du courant alternatif.

6. Appareil réfrigérant et chauffant selon l'une des revendications précédentes, **caractérisé en ce que** l'élément échangeur de chaleur (17) est relié aux conduites de fluide (16) par l'intermédiaire de raccords rapides (18).

7. Appareil réfrigérant et chauffant selon l'une des revendications précédentes, **caractérisé en ce que** les conduites de fluide (16) sont dotées, du côté du réservoir, de raccords rapides (18).

8. Appareil réfrigérant et chauffant selon l'une des revendications précédentes, **caractérisé en ce que** le réservoir (6) est relié à un tube de niveau de remplissage (12), permettant d'indiquer le niveau du milieu dans le réservoir (6).

9. Appareil réfrigérant et chauffant selon l'une des revendications précédentes, **caractérisé en ce qu'**il est prévu, dans ou sur le réservoir (6), une unité d'affichage qui présente par exemple des DEL (21) pour afficher le niveau du milieu dans le réservoir (6).

10. Appareil réfrigérant et chauffant selon l'une des revendications précédentes, **caractérisé en ce que** le réservoir (6) et/ou les conduites de fluide (16) sont dotés au moins en partie d'une isolation thermique.

11. Appareil réfrigérant et chauffant selon l'une des revendications précédentes, **caractérisé en ce que** le dispositif de commande (10) comporte un clavier (19) permettant d'entrer les paramètres de fonctionnement.

12. Appareil réfrigérant et chauffant selon l'une des revendications précédentes, **caractérisé en ce qu'**un dispositif d'affichage (20), en particulier alphanumérique, est associé au dispositif de commande (10) afin d'afficher les paramètres de fonctionnement.

13. Appareil réfrigérant et chauffant selon l'une des revendications précédentes, **caractérisé en ce que** l'élément échangeur de chaleur est par exemple réalisé sous la forme d'une manchette (17) qui a une forme pouvant être adaptée en particulier aux parties corporelles d'un patient.

14. Appareil réfrigérant et chauffant selon l'une des revendications précédentes, **caractérisé en ce que** l'élément échangeur de chaleur (17) comporte une première chambre qui est reliée à la pompe (7) par l'intermédiaire de conduites de fluide (16), et une autre chambre qui est reliée à l'autre dispositif de pompe (25) par l'intermédiaire d'une conduite (22).

15. Appareil réfrigérant et chauffant selon l'une des revendications précédentes, **caractérisé en ce que** le débit du dispositif de pompe (25) peut être réglé indépendamment du débit de la pompe (7).

16. Appareil réfrigérant et chauffant selon l'une des revendications précédentes, **caractérisé en ce que** le dispositif de pompe (25) génère une pression allant jusqu'à 200 mbars dans l'élément échangeur de chaleur (17).

17. Appareil réfrigérant et chauffant selon l'une des revendications précédentes, **caractérisé en ce qu'**au moins un ventilateur (8) pouvant être réglé, en particulier par l'intermédiaire du dispositif de commande (10), est associé à la source et au dissipateur de chaleur (9).
